# EUROPEAN PATENT SPECIFICATION

(11) **EP 0 972 846 B1**
(45) Date of publication and mention of the grant of the patent: **17.11.2004**
(21) Application number: 99111749.0
(22) Date of filing: 17.06.1999
(51) Int. Cl.: C12Q 1/04, C12M 1/00

(54) **Searching and detecting microorganisms**
Auffinden und Nachweisen von Mikroorganismen
Recherche et détection de microorganismes

(30) Priority: 17.06.1998 IT MO980133
(43) Date of publication of application: 19.01.2000
(73) Proprietor: Murgia, Sergio, 09012 Capoterra (CA) (IT)
(72) Inventor: Murgia, Sergio, 09012 Capoterra (CA) (IT)
(74) Representative: Luppi, Luigi

(56) References cited:
- WO-A-92/06213
- WO-A-96/00780
- US-A- 4 318 994

## Description

The invention relates to the field of devices for searching for and detecting microorganisms into culture mediums. The microaerophilic microorganisms contained in a sample grow on a suitable culture medium in an environment containing an atmosphere with a small quantity of oxygen and a CO₂ amount from 5% to 10%.

The prior art includes different systems for generating such an environment that is then kept by subjecting the culture ground to an incubation process.

A first method provides for cylindrical containers of Plexiglas being used, in which the desired environment is created using a compound initially contained in small envelopes that, once hydrated, reacts producing CO₂ and an atmosphere with a small quantity of oxygen.

In a second method tablets are used which are introduced in a closed system containing the culture medium.

In both the cases, the system for generating the growth environment of microaerophilic microorganisms in a research apparatus prevents a growing of aerobic microorganisms from being achieved in the same apparatus.

US 4318994 discloses a device consisting of a plastic plate in which a plurality of wells is made, said wells being connected, by means of a serpentine channel, with an adjacent portion of feeder channels which communicate with a filler port. Opposite surfaces of the plate are sealed by means of a layer of gas-permeable transparent tape. Each well contains a suitable culture medium and it is also provided with a duct debouching into an overflow chamber. In use, a liquid sample containing a microorganism to be identified is injected into the filler port. In this way, the liquid sample fills each well, prevention and elimination of bubbles being provided by overflow chambers. If a microaerophilic environment is needed in some wells, further layers of transparent tape can be added in order to reduce the amount of oxygen that can pass through the tape. A disadvantage of the device disclosed in US 4318994 is that no effective means is provided to generate a microaerophilic environment in the wells.

WO 9600780 discloses a device for cell culture that can be used in a CO₂ incubator. The device substantially consists of a cell culture compartment including a lower gas permeable film, and an upper dialysis membrane, through which the cell culture compartment is made communicating with a basal medium compartment. In use, a liquid medium containing cells to be cultured is introduced into the cell culture compartment, while a liquid basal medium is introduced into the basal medium compartment for a continuous dialysis of the culture. CO₂ can penetrate in the cell culture compartment, allowing pericellular pH to be maintained at suitable level. A disadvantage of the device disclosed in WO 9600780 is that it provides a cell culture compartment in which all the cells are subject to a same gaseous environment comprising CO₂.

It is a purpose of the present invention to improve the known devices for searching of the above microorganisms.

More in particular, it is desirable to make available integrated, possibly miniaturized environments for the growth of microorganisms with different atmospheres.

It is a further purpose of the present invention to realize a system for carrying out the growth both of microaerophilic microorganisms and of aerobic microorganisms into a same support.

In a first aspect of the present invention, there is provided an apparatus for searching microorganism, comprising a container having well means capable of containing a culture medium, characterized in that it further comprises cell means containing a composition designed to generate a gaseous mass, and duct means connecting said cell means to said well means.

In an particularly advantageous embodiment the gaseous mass comprises an atmosphere with a small quantity of oxygen, enriched with a quantity of 5% - 10% of CO₂.

The well means can comprise at least one well containing a culture medium for microaerophilic microorganisms and at least one well containing a culture medium for aerobic microorganisms.

In a particularly advantageous version, the apparatus comprises sealing means capable of sealing the cell means to prevent CO₂ losses and entry of oxygen into the wells containing a culture medium for microaerophilic microorganisms so that an environment with an appropriate percentage of such a gas may be maintained.

On the contrary, the wells containing a culture medium for aerobic microorganisms are left open.

In such a manner, the growth both of microaerophilic microorganisms and aerobic microorganisms is carried out in a same apparatus.

In a preferred version, the wells exhibit a rectangular shape in plan view.

In a second aspect of the present invention, there is provided a method for searching microorganism, comprising:
- preparing a composition designed to generate a gaseous mass;
- introducing said composition into cell means shaped as tablets, capsules, or gel.
- activating said composition for generating said gaseous mass;
- transferring, said gaseous mass through duct means into well means comprising at least one well containing a culture medium for the growth of microaerophilic microorganisms.
- sealing said cell means and said at least one well.

In a particularly advantageous version the prepared composition comprises citric acid, sodium bicarbonate, or sodium carbonate, iron powder and an inert material that can be siliceous clay.

The composition to be introduced into the cell means is advantageously contained in a tablet, or in a capsule, or in a gel, with a suitable dispenser, for being more easily introduced.

The activation of the composition is obtained preferably by supplying water, advantageously an amount from about 50 µl to about 200 µl, preferably 100 µl.

Sealing of the cell means and the wells containing a culture medium for microaerophilic microorganisms, is obtained preferably by applying an adhesive tape, preferably transparent and oxygen proof.

In a further advantageously version, a certain percentage of water percentage is contained in the cell means, the water being for example incorporated in a porous base impregnated with water or in any way hydrated, such a base advantageously comprising, for example, a hydrated gel, for example obtained from agar (in suggested proportions comprised between 4 and 7 g/l) by adding an appropriate amount of water.

In such a way, when the compound is introduced into the cell means its activation is automatic, because the water contained in the base hydrated gel causes the activation of the composition.

In a further version, the well means and/or the cell means contains detector means capable of detecting the presence of oxygen, said means, when the composition has been activated and the sealing has been carried out, gives indications about the insulated environment in order to signal when the oxygen presence exceeds an admissible limit.

As detection means methylene blue may be employed.

The detection means may be associated with the base, or may be associated with the adhesive tape means for sealing.

The invention will be better understood and carried out with reference to the attached drawings, that illustrate some nonlimiting embodiments thereof, wherein:
Figure 1 is a plan view of an apparatus for searching microorganisms according the invention;
Figure 2 is the section II-II of Figure 1;
Figure 3 is a view as in Figure 1, but concerning a further version of apparatus according to the invention;
Figure 4 is the section IV-IV of Figure 3;
Figure 5 is a perspective view of a further version of the apparatus.

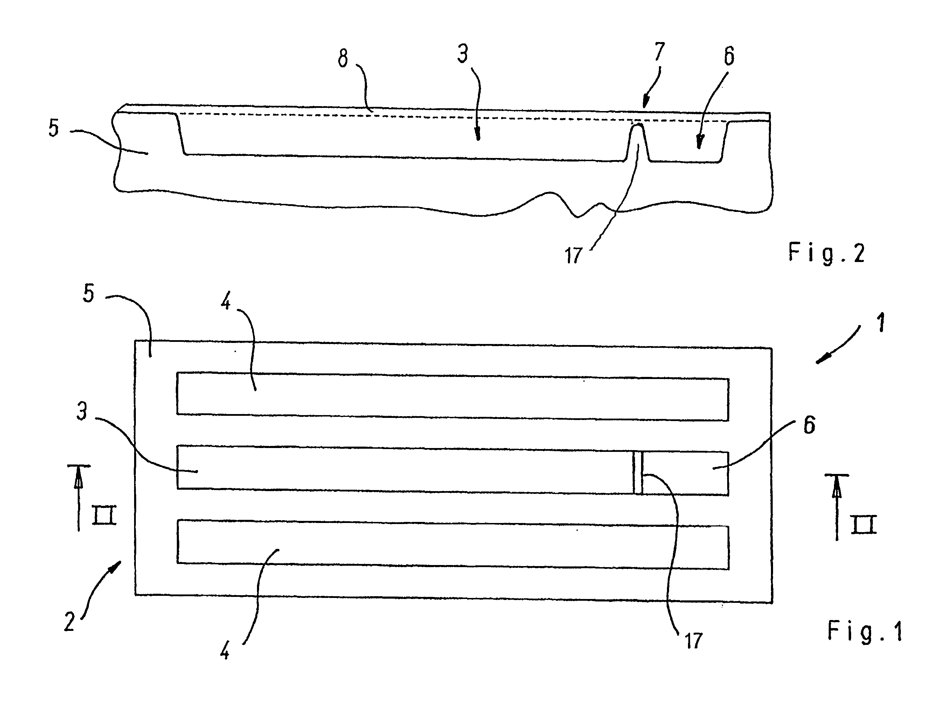

With reference to Figures 1 and 2, an apparatus 1 for the growth of both microaerophilic microorganisms and aerobic microorganisms, comprises a container 5 provided with well means 2 containing a culture medium. The well means 2 includes at least a well 3 designed to contain a culture medium for the growth of microaerophilic microorganisms and at least a further well 4 designed to contain a culture medium for the growth of aerobic microorganisms. The wells 3 and 4 exhibit preferably a rectangular shape in a plan view. The container 5 is further provided with cell means 6, for example having a rectangular, or circular, shape, to which duct means 7 is coupled that connects the cell means 6 with the well 3. The cell means 6 is designed to receive a composition containing preferably citric acid, sodium bicarbonate, or sodium carbonate, iron powder and an inert material, for example siliceous clay, that is prepared separately. This composition may be in the form of powder, or pomade (gel); it is anyway preferable to enclose it in a tablet, in order to make easier to introduce it into the cell means 6. The composition, after being supplied with water, develops a reaction that produces CO₂· that can reach the well 3 through the duct means 7 and reduces the oxygen percentage, because a part thereof links with iron powder, forming iron oxide. Sealing means 8 (Figure 2), for example comprising adhesive tape, may be used for hermetically closing the cell means 6 and the wells 3 for the growth of microaerophilic microorganisms and for maintaining an environment having a suitable percentage of CO₂. On the contrary, the further wells 4 are kept open in order to permit the growth of aerobic microorganisms.

The duct means 7 is defined between a partition 17, separating the cell means 6 from the well means 3, and the sealing sheet means 8 that is applied to close the upper opening of the cell means 6 and the well means 3.

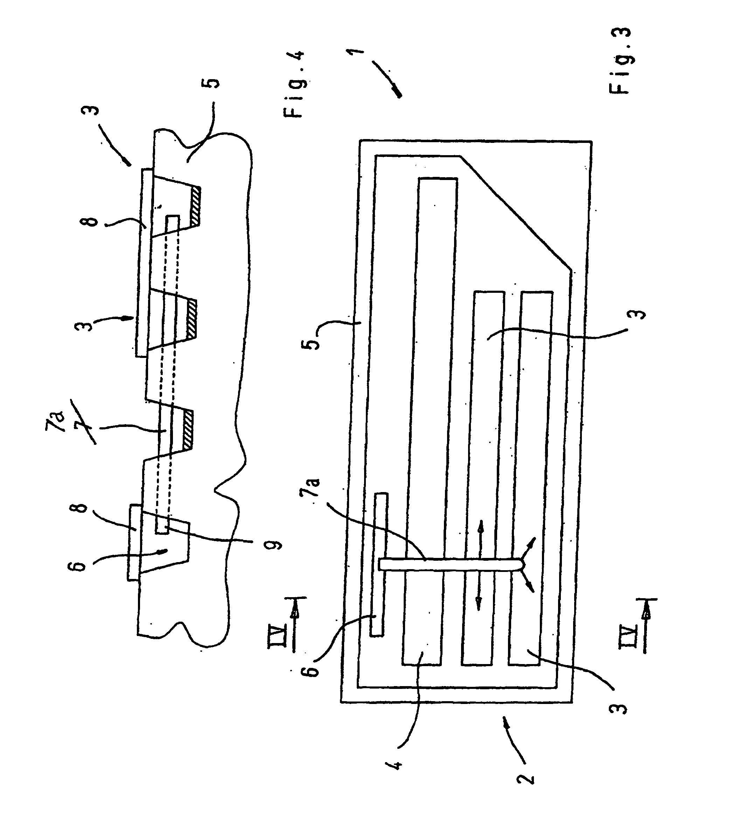

The version of Figures 3 and 4 differs from the version described because it has only one cell 6 in the container 5 and the duct means including a tube 7a, having an end 9 connected with the cell 6 and extending in a transversal direction of the container 5, so as to pass through the various wells 3 and 4.

Instead of the tube 7a a groove can be provided disposed so as to make the well means 2 and 3 communicating between themselves under the sealing means 8 that closes them upwardly. In this case, however, the wells are all designed to occupy an environment substantially free from oxygen.

In the region of the wells 3 for the growth of microaerophilic microorganisms, the tube 7a is provided with openings for introducing CO₂ into the wells and for allowing passage of oxygen that reacts with the iron powder. The tube 7 is closed in the region of the further well 4 for the growth of aerobic microorganisms. Sealing with the adhesive tape 8 is carried out in the wells 3 and in the cell 6. In this version, therefore, a single cell 6 is sufficient for each container 5 provided with a plurality of wells 3 for the growth of microaerophilic microorganisms, whereas in the version of Figures 1 and 2, each well 3 is associated to a respective cell 6.

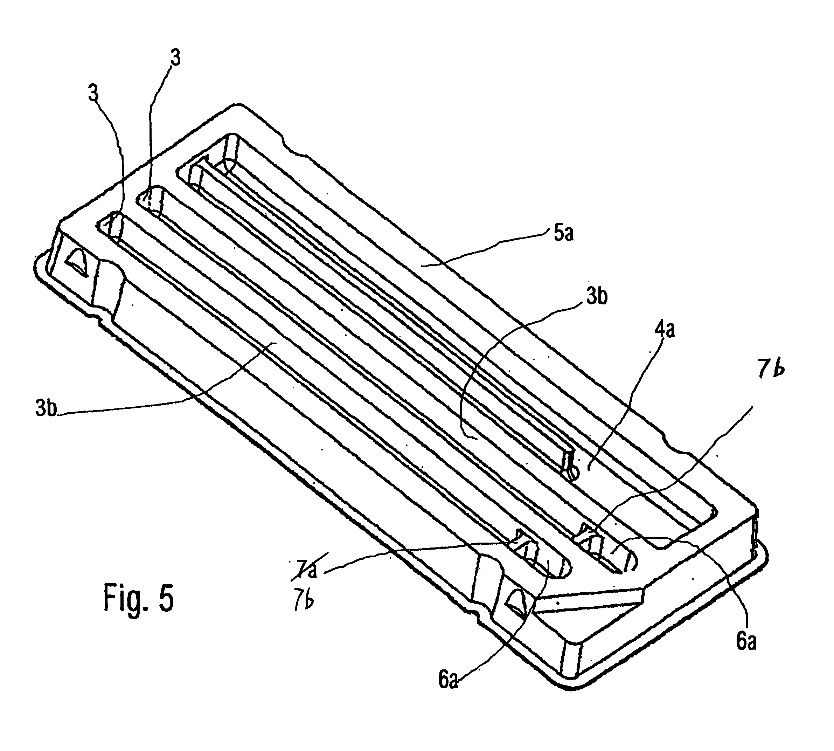

As shown in Figure 5, a container 5a obtained by thermoforming a plastic material, is provided with well means 4a kept in vacuum state and showing a U arrangement in plan view , said means being of the type disclosed in European Patent application n. 98119429.3, and with analogous rectilinear well means 3 adjoining cell means 6a and separated therefrom by wall means 7b shorter than the side walls 3b of the well means 3 and cell means 6a. In this case, the duct means is defined in the gap between the wall means and the sealing sheet 8.

## Claims

1. Apparatus for searching microorganisms, comprising a container (5) provided with well means (2) containing a culture medium, cell means (6) and duct means (7, 7a) connecting said cell means (6) with said well means (2) **characterized in that** said cell means (6) contains a composition designed to generate a gaseous mass.

2. Apparatus according to claim 1, wherein said well means (2) comprises at least one well (3) containing a culture medium for microaerophilic microorganisms and at least a further well (4) containing a culture medium for aerobic microorganisms.

3. Apparatus according to claim 1, or 2, and further comprising sealing means (8) capable of sealing said at least one well (3) containing a culture medium for microaerophilic microorganisms.

4. Apparatus according to claim 3, wherein said sealing means comprises adhesive tape (8).

5. Apparatus according to any preceding claim, wherein said composition comprises citric acid, sodium bicarbonate, or sodium carbonate, and iron powder.

6. Apparatus according to any preceding claim, wherein said gaseous mass comprises CO₂.

7. Apparatus according to claim 6, wherein said gaseous mass comprises a volume percentage of CO₂ comprised between about 5% and 10% and a volume percentage of oxygen comprised between about 5% and 7%.

8. Apparatus according to claim 4, wherein said composition is capable of being enclosed in a tablet, or in a capsule, or in a gel composition.

9. Apparatus according to any preceding claim, wherein said cell means (6) are capable of receiving a substance suitable for causing a reaction of said mixture designed to produce CO₂ and chemically linking itself with oxygen.

10. Apparatus according to one of claims 1 to 8, wherein said cell means (6) contains a substance capable of causing a reaction of said composition designed to produce CO₂ and chemically linking itself with oxygen.

11. Apparatus according to claim 9, or 10, wherein said substance comprises water.

12. Apparatus according to one of claims 9 to 11, wherein said substance comprises an amount of water comprised between about 50 µl and 200 µl.

13. Apparatus according to claim 12, wherein said amount is about 100 µl.

14. Apparatus according to any preceding claim, wherein said well means (2) has a rectangular shape in plan view.

15. Apparatus according to any preceding claim, wherein said cell means (6) are shared by a plurality of said well means (2).

16. Apparatus according to one of claims 3 to 15, wherein said duct means (7) is defined by a passage comprised between said sealing means (8) and wall means (17, 7a) that separates said well means (3) from said cell means (6).

17. Apparatus according to one of claims 1 to 15, wherein said duct means (7) comprises a tube (7) that passes through said well means (2).

18. Apparatus according to claim 17, wherein said tube (7) is provided with openings in the region of said at least one well (3) containing a culture medium for microaerophilic microorganisms.

19. Apparatus according to any preceding claim, wherein said well means (3) and/or said cell means (6) contains detector means capable of indicating the presence of oxygen when said sealing means has been applied.

20. Apparatus according to claim 19, wherein said detection means is applied to said sealing means.

21. Method for searching microorganisms comprising:
- preparing a composition designed to generate a gaseous mass;
- introducing said composition into cell means (6);
- activating said composition for generating said gaseous mass;
- transferring, through duct means (7), said gaseous mass into well means (2) comprising at least one well (3) containing a culture medium for the growth of microaerophilic microorganisms.
- sealing said cell means (6) and said at least one well (3)
**characterized in that** said duct means (7) extends between said cell means (6) and said at least one well (3).

22. Method according to claim 21, wherein said preparing comprises preparing a composition comprising citric acid, sodium bicarbonate, or sodium carbonate, and iron powder.

23. Method according to claim 21, o 22, and further comprising, enclosing said composition in a tablet before said introducing.

24. Method according to claim 21, o 22, wherein said activating comprises activating said composition for generating CO₂ and binding oxygen.

25. Method according to one of claims 21 to 24, wherein said activating comprises supplying water.

26. Method according to claim 25, wherein said supplying comprises supplying a water amount comprised between about 50 ml and 200 ml.

27. Method according to claim 26, wherein said amount is about 100 ml.

28. Method according to one of claims 21 to 27, wherein said sealing comprises applying adhesive tape (8).

29. Method according to one of claims 21 to 28, wherein it is provided for liberating oxygen detector means in said well means (3) and/or in said cell means (6) before said sealing.

## Patentansprüche

1. Vorrichtung zum Suchen von Mikroorganismen, mit einem Behälter (5), der ein Schachtmittel (12) aufweist, das ein Kulturmedium enthält, ein Zellenmittel (6) und Kanalmittel (7, 7a), um das Zellenmittel (6) mit dem Schachtmittel (2) zu verbinden, **dadurch gekennzeichnet, dass** das Zellenmittel (6) eine Zusammensetzung enthält, die zur Erzeugung einer gasförmige Masse ausgebildet ist.

2. Vorrichtung nach Anspruch 1, bei der das Schachtmittel (2) wenigstens einen Schacht (3) aufweist, der ein Kulturmedium für mikroaerophile Mikroorganismen und wenigstens einen weiteren Schacht (4), der ein Kulturmedium für aerobe Mikroorganismen enthält, aufweist.

3. Vorrichtung nach Anspruch 1 oder 2, die ferner ein Dichtungsmittel (8) aufweist, das zum Abdichten des wenigstens einen Schachtes (3), der ein Kulturmedium für mikroaerophile Mikroorganismen enthält, geeignet ist.

4. Vorrichtung nach Anspruch 3, bei der das Dichtmittel Klebeband (8) enthält.

5. Vorrichtung nach irgendeinem der vorhergehenden Ansprüche, bei der die genannte Zusammensetzung Zitronensäure, doppeltkohlensaures Natrium oder Natriumcarbonat und Eisenpulver enthält.

6. Vorrichtung nach irgendeinem der vorhergehenden Ansprüche, bei der die gasförmige Masse CO₂ enthält.

7. Vorrichtung nach Anspruch 6, bei der die gasförmige Masse einen Volumenanteil an CO₂ enthält, der zwischen ungefähr 5 und 10 % eines Volumenprozentsatzes von Sauerstoff liegt, der zwischen 5 und 7 % liegt.

8. Vorrichtung nach Anspruch 4, bei der die genannte Zusammensetzung zum Einschluss in einer Tablette oder in einer Kapsel oder in einer Gelzusammensetzung geeignet ist.

9. Vorrichtung nach irgendeinem der vorhergehenden Ansprüche, bei der die Zellenmittel (6) zur Aufnahme einer Substanz geeignet sind, die zur Erzeugung einer Reaktion der zur Erzeugung von CO₂ geeigneten Mischung geeignet ist und chemisch selbst mit Sauerstoff verbunden ist.

10. Vorrichtung nach einem der Ansprüche 1 bis 8, bei der das Zellenmittel (6) eine Substanz enthält, die zur Erzeugung einer Reaktion der genannten zur Erzeugung von CO₂ geeigneten Zusammensetzung geeignet ist und chemisch selbst mit Sauerstoff verbunden ist.

11. Vorrichtung nach Anspruch 9 oder 10, bei der die genannte Substanz Wasser enthält.

12. Vorrichtung nach irgendeinem der Ansprüche 9 bis 11, bei der die genannte Substanz eine Menge von Wasser enthält, die zwischen ungefähr 50 µl und 200 µl liegt.

13. Vorrichtung nach Anspruch 12, bei der die Menge ungefähr 100 µl ist.

14. Vorrichtung nach irgendeinem der vorhergehenden Ansprüche, bei der das Schachtmittel (2) in der ebenen Ansicht eine rechteckförmige Form aufweist.

15. Vorrichtung nach irgendeinem der vorhergehenden Ansprüche, bei der die Zellenmittel (6) von einer Mehrzahl der Schachtmittel (2) geteilt werden.

16. Vorrichtung nach einem der Ansprüche 3 bis 15, bei der das Kanalmittel (7) von einem Durchlass definiert ist, der zwischen dem Dichtmittel (8) und dem Wandmittel (17, 7a) enthalten ist, das das Schachtmittel (3) von dem Zellmittel (6) trennt.

17. Vorrichtung nach einem der Ansprüche 1 bis 15, bei der das Kanalmittel (7) ein Rohr (7) aufweist, das sich durch das Schachtmittel (2) erstreckt.

18. Vorrichtung nach Anspruch 17, bei der das Rohr (7) mit Öffnungen in dem Bereich des wenigstens einen Schachtmittels (3) versehen ist, das ein Kulturmedium für mikroaerophile Mikroorganismen enthält.

19. Vorrichtung nach irgendeinem der vorhergehenden Ansprüche, bei der das Schachtmittel (3) und/oder das Zellenmittel (6) ein Detektormittel enthält, das zur Anzeige des Vorhandenseins von Sauerstoff geeignet ist, wenn das Dichtmittel aufgebracht wurde.

20. Vorrichtung nach Anspruch 19, bei der das Detektionsmittel auf das Dichtmittel aufgebracht wird.

21. Verfahren zum Suchen von Mikroorganismen mit folgenden Schritten:
- Bereitstellen einer Zusammensetzung, die zur Erzeugung einer gasförmigen Masse geeignet ist;
- Einführen der Zusammensetzung in ein Zellenmittel (6);
- Aktivieren der Zusammensetzung zur Erzeugung der gasförmigen Masse;
- Übertragen der gasförmigen Masse durch ein Kanalmittel (7) in ein Schachtmittel (2), das wenigstens einen Schacht (3) enthält, der ein Kulturmedium für das Wachstum von mikroaerophilen Mikroorganismen aufweist;
- Abdichten des Zellenmittels (6) und des wenigstens einen Schachtes (3);
**dadurch gekennzeichnet, dass** sich das Kanalmittel (7) zwischen dem Zellenmittel (6) und dem wenigstens einen Schacht (3) erstreckt.

22. Verfahren nach Anspruch 21, bei dem das Bereitstellen das Bereitstellen einer Zusammensetzung aufweist, die Zitronensäure, doppeltkohlensaures Natrium oder Natriumcarbonat und Eisenpulver enthält.

23. Verfahren nach Anspruch 21 oder 22, das ferner den Schritt des Einschließens der Zusammensetzung in einer Tablette vor dem Einführen enthält.

24. Verfahren nach Anspruch 21 oder 22, bei dem das Aktivieren das Aktivieren der Zusammensetzung zur Erzeugung von CO₂ und von bindendem Sauerstoff aufweist.

25. Verfahren nach einem der Ansprüche 21 bis 24, bei dem das Aktivieren das Bereitstellen von Wasser aufweist.

26. Verfahren nach Anspruch 25, bei dem das Bereitstellen das Bereitstellen von Wasser in einer Menge zwischen ungefähr 50 ml und 200 ml aufweist.

27. Verfahren nach Anspruch 26, bei dem die Menge ungefähr 100 ml ist.

28. Verfahren nach einem der Ansprüche 21 bis 27, bei dem das Abdichten das Aufbringen von Klebeband (8) enthält.

29. Verfahren nach einem der Ansprüche 21 bis 28, bei dem Vorsorge getroffen ist zur Freisetzung eines Sauerstoffdetektormittels in dem Schachtmittel (3) und/oder in dem Zellenmittel (6) vor dem Abdichten.

## Revendications

1. Dispositif pour la recherche de microorganismes, comprenant un récipient (5) muni de moyens de puits (2) contenant un milieu de culture, des moyens de cellule (6) et des moyens de canal (7, 7a) reliant lesdits moyens de cellule (6) avec lesdits moyens de puits (2), ***caractérisé en ce que*** lesdits moyens de cellule (6) contiennent une composition conçue pour produire une masse gazeuse.

2. Dispositif selon la revendication 1, dans lequel lesdits moyens de puits (2) comprennent au moins un puits (3) contenant un milieu de culture pour des microorganismes micro-aérophiles et au moins un puits supplémentaire (4) contenant un milieu de culture pour des microorganismes aérobies.

3. Dispositif selon la revendication 1 ou 2, comprenant de plus des moyens d'obturation (8) aptes à obturer au moins un puits (3) contenant un milieu de culture pour des microorganismes micro-aérophiles.

4. Dispositif selon la revendication 3, dans lequel lesdits moyens d'obturation comprennent une bande adhésive (8).

5. Dispositif selon l'une quelconque des revendications précédentes, dans lequel ladite composition comprend de l'acide citrique, du bicarbonate de sodium ou du carbonate de sodium, et de la poudre de fer.

6. Dispositif selon l'une quelconque des revendications précédentes, dans lequel ladite masse gazeuse comprend du CO₂.

7. Dispositif selon la revendication 6, dans lequel ladite masse gazeuse comprend un pourcentage volumique de CO₂ compris entre environ 5 % et 10 % et un pourcentage volumique d'oxygène compris entre environ 5 % et 7 %.

8. Dispositif selon la revendication 4, dans lequel ladite composition est apte à être incluse dans un cachet, ou dans une capsule, ou dans une composition sous forme de gel.

9. Dispositif selon l'une quelconque des revendications précédentes, dans lequel lesdits moyens de cellule (6) sont aptes à recevoir une substance propre à provoquer une réaction dudit mélange pour produire du CO₂ et à se lier chimiquement à l'oxygène.

10. Dispositif selon l'une des revendications 1 à 8, dans lequel lesdits moyens de cellule (6) contiennent une substance apte à provoquer une réaction de ladite composition destinée à produire du CO₂ et à se lier chimiquement à l'oxygène.

11. Dispositif selon la revendication 9 ou 10, dans lequel ladite substance comprend de l'eau.

12. Dispositif selon l'une des revendications 9 à 11, dans lequel ladite substance comprend une quantité d'eau comprise entre 50 µl et 200 µl environ.

13. Dispositif selon la revendication 12, dans lequel ladite quantité est d'environ 100 µl.

14. Dispositif selon l'une quelconque des revendications précédentes, dans lequel lesdits moyens de puits (2) ont une forme rectangulaire selon une vue en plan.

15. Dispositif selon l'une quelconque des revendications précédentes, dans lequel lesdits moyens de cellule (6) sont partagés par une pluralité desdits moyens de puits (2).

16. Dispositif selon l'une des revendications 3 à 15, dans lequel lesdits moyens de canal (7) sont définis par un passage compris entre lesdits moyens d'obturation (8) et des moyens de paroi (17, 7a) qui séparent lesdits moyens de puits (3) desdits moyens de cellule (6).

17. Dispositif selon l'une des revendications 1 à 15, dans lequel lesdits moyens de canal (7) comprennent un tube (7) qui traverse lesdits moyens de puits (3).

18. Dispositif selon la revendication 17, dans lequel ledit tube (7) est équipé d'ouvertures dans la région dudit au moins un puits (3) contenant un milieu de culture pour des microorganismes micro-aérophiles.

19. Dispositif selon l'une quelconque des revendications précédentes, dans lequel lesdits moyens de puits (3) et/ou lesdits moyens de cellule (6) contiennent des moyens de détection aptes à indiquer la présence d'oxygène lorsque lesdits moyens d'obturation ont été appliqués.

20. Dispositif selon la revendication 19, dans lequel lesdits moyens de détection sont appliqués sur lesdits moyens d'obturation.

21. Procédé de recherche de microorganismes comprenant les étapes de :
- préparation d'une composition conçue pour produire une masse gazeuse ;
- introduction de ladite composition dans un moyen de cellule (6) ;
- activation de ladite composition pour produire ladite masse gazeuse ;
- transfert, à travers des moyens de canal (7), de ladite masse gazeuse dans des moyens de puits (2) comprenant au moins un puits (3) contenant un milieu de culture pour la croissance de microorganismes micro-aérophiles ;
- obturation desdits moyens de cellule (6) et dudit au moins un puits (3) ;
***caractérisé en ce que*** lesdits moyens de canal (7) s'étendent entre lesdits moyens de cellule (6) et ledit au moins un puits (3).

22. Procédé selon la revendication 21, dans lequel ladite préparation comprend la préparation d'une composition comprenant de l'acide citrique, du bicarbonate de sodium ou du carbonate de sodium, et de la poudre de fer.

23. Procédé selon la revendication 21 ou 22, et comprenant de plus l'inclusion de ladite composition dans un cachet avant ladite introduction.

24. Procédé selon la revendication 21 ou 22, dans lequel ladite activation comprend l'activation de ladite composition pour produire du CO₂ et se lier à l'oxygène.

25. Procédé selon l'une des revendications 21 à 24, dans lequel l'activation comprend un apport d'eau.

26. Procédé selon la revendication 25, dans lequel ledit apport comprend l'apport d'une quantité d'eau comprise entre 50 ml et 200 ml environ.

27. Procédé selon la revendication 26, dans lequel ladite quantité est d'environ 100 ml.

28. Procédé selon l'une des revendications 21 à 27, dans lequel ladite obturation comprend l'application d'une bande adhésive (8).

29. Procédé selon l'une des revendications 21 à 28, dans lequel il est prévu la libération de moyens de détection de l'oxygène dans lesdits moyens de puits (3) et / ou dans lesdits moyens de cellule (6) avant ladite obturation.
